(19) Europäisches Patentamt / European Patent Office / Office européen des brevets

(11) **EP 3 157 539 B1**

(12) **EUROPEAN PATENT SPECIFICATION**

(45) Date of publication and mention of the grant of the patent:
**22.05.2019 Bulletin 2019/21**

(21) Application number: **15809840.0**

(22) Date of filing: **16.06.2015**

(51) Int Cl.:
*A61K 36/064* *(2006.01)*    *A61K 36/07* *(2006.01)*
*C12N 1/16* *(2006.01)*    *C12N 1/22* *(2006.01)*
*A23K 10/12* *(2016.01)*    *A23K 10/32* *(2016.01)*
*A23L 33/135* *(2016.01)*

(86) International application number:
**PCT/FI2015/050438**

(87) International publication number:
**WO 2015/193548 (23.12.2015 Gazette 2015/51)**

(54) **USE OF HYDROTHERMALLY TREATED BIOMASS AS PATHOGEN BINDER**

VERWENDUNG VON HYDROTHERMISCH BEHANDELTER BIOMASSE ALS PATHOGENBINDER

UTILISATION DE BIOMASSE TRAITÉE DE FAÇON HYDROTHERMIQUE EN TANT QUE LIANT D'AGENT PATHOGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2014 EP 14172852**

(43) Date of publication of application:
**26.04.2017 Bulletin 2017/17**

(73) Proprietor: **Neste Oyj**
**02150 Espoo (FI)**

(72) Inventors:
• **PENNALA, Eero**
**FI-02110 Espoo (FI)**
• **APAJALAHTI, Juha**
**FI-00670 Helsinki (FI)**
• **MALM, Annika**
**FI-00510 Helsinki (FI)**
• **PASANEN, Jukka-Pekka**
**FI-06101 Porvoo (FI)**

• **HEISKA, Arto**
**FI-00710 Helsinki (FI)**

(74) Representative: **Zacco Denmark A/S**
**Arne Jacobsens Allé 15**
**2300 Copenhagen S (DK)**

(56) References cited:
EP-A1- 2 450 424    EP-A1- 2 450 424
EP-A1- 2 450 425    EP-A1- 2 450 425
WO-A1-2010/030197    WO-A1-2010/030197
WO-A1-2014/188005    WO-A2-2004/044215
US-A1- 2008 268 096    US-A1- 2008 268 096
US-A1- 2010 330 615    US-A1- 2010 330 615
US-A1- 2012 027 747    US-A1- 2012 082 759
US-A1- 2012 082 759

• KOGAN G. ET AL.: 'Role of yeast cell wall polysaccharides in pig nutrition and health protection.' LIVESTOCK SCIENCE vol. 109, no. 1-3, 2007, ISSN 1871-1413 pages 161 - 165, XP022053296

## Description

### Field of the invention

The present invention relates to microorganism biomass, or a feed or food composition comprising said biomass for use in preventing or reducing the adverse effects of pathogens in animal or human digestive tract. The invention is strictly defined by the claims.

### Background of the invention

The digestive tract of human and animals may be infected by many pathogens. In human this causes disorders in digestive tract or illnesses, in animals this causes economic losses due to lowered productivity, illnesses or death. In addition, pathogens may cause food borne infections for consumers, if entered into the food chain. Many of the pathogens are part of the normal intestinal flora, and illness may be triggered for example by stress.

Pathogenic *Salmonella enterica* strains are a distressing health problem both with humans and animals worldwide. S. *enterica* serotype Choleraesuis shows the highest tendency to cause systemic infections. Emergence of S. *enterica* strains that are resistant to chloramphenicol and other antibiotics has aroused concern about the use of these agents for the treatment of systemic infection caused by *Salmonella. Salmonella enterica* is a facultatively anaerobic Gram-negative bacterium.

*Escherichia coli* is also part of the normal microbiota of animal or humans. Some *E. coli* serotypes are harmful, e.g. *E. coli* F4+. *E. coli* are the major cause of weaning diarrhea in piglets. *Escherichia coli* is a Gram-negative bacterium.

Kogan and Kocher (2007.Role of yeast polysaccharides in pig nutrition and health protection, Livestock science, 109 (2007)161-165) describe that certain yeast cell wall polysaccharides were able to block fimbriae of pathogenic bacteria and thus prevent their adhesion to the mucous epithelium. The bacterial lectin binding capacity of one commercial yeast wall polysaccharide preparation Bio-Mos® (Alltech, Inc.) was tested with agglutination tests with 258 pathogenic strains of four bacterial genera.

Additionally, the binding of pathogens has been reported by using different adsorbents, for example activated charcoal (Knutson et al. 2006. Effects of activated charcoal on binding E. coli O157:H7 and Salmonella typhimurium in sheep, Small Ruminant Res. 65 (2006) 101-105).

US 2012/0034344A1 (Menon et al) describes a method for producing lipids from lignocellulosic feedstock by fermentation. In the method, bacterial and /or fungal strains, such as *Trichoderma reesei,* were used to convert cellulose, hemicellulose or glycerol into lipids and fungus of the genus *Penicillium* was used to degrade cellulose into sugars, which could be used by the lipid producing microbes. In the method described in the publication fungal biomass depleted of a liquid product was used for animal meal.

US 2012/0082759 relates to the use of dry yeast biomass for mycotoxin binding showing various degrees of toxin binding.

Although some attempts to eliminate or reduce the effects of pathogens in digestive tract are known from the prior art, there is still a need for efficient products and methods which could be used against pathogens in the digestive tract of human and animals.

### Summary of the invention

One object of the present invention is to provide a solution to the problems encountered in the prior art.

In particular, it is one object of the present invention to provide a solution, which enables prevention or reduction of the adverse effects of pathogens in animal or human digestive tract.

To achieve these objects the invention is characterized by the features that are enlisted in the independent claims. Other claims represent the preferred embodiments of the invention.

The invention is based on the finding that microbial biomass prevents and reduces the adverse effects of pathogens in animal or human digestive tract.

It has now been surprisingly found that microbial biomass, in particular biomass, which has been obtained by cultivating microorganisms on a cultivation medium comprising lignocellulosic material has this ability of preventing and reducing the adverse effects of pathogens.

Moreover, it has been surprisingly found that a particularly effective pathogen binding is observed when the microbial biomass is processed hydrothermally with a severity factor of about 3.5 to about 5.5 such as e.g. about 4 to about 4.9, such as e.g. about 4.1 to about 4.8.

Accordingly, a method for preparing a feed or food additive is described, said method comprising the steps of

(a) providing an aqueous suspension comprising microbial biomass,

(b) subjecting said aqueous suspension comprising said microbial biomass to a temperature of at least 160°C for 1s to 360 minutes at a pressure above 5 bar,

(c) separating a solid phase containing the microbial biomass,

(d) optionally, drying the microbial biomass obtained from step (c).

The present invention relates to a method for inactivating a pathogen in a contaminated feed or food product, said method comprising the steps of

(a) providing a feed or food product contaminated with a pathogen,

(b) providing a feed or food additive of the present invention,

(c) mixing the feed or food product of (a) with the feed or food additive of (b).

Further described is a method for preparing a feed or food product, said method comprising the steps of

(a) providing a feed or food substance,

(b) providing a feed or food additive of the present invention,

(c) mixing the feed or food substance of (a) with the feed or food additive of (b) to obtain a feed or food product.

A fifth aspect of the present invention relates to a feed product obtainable from any of the preceding method for a method for preparing a feed or food product.

Also described is a method for feeding an animal with a feed product of the present invention.

One aspect of the present invention provides hydrothermally processed microbial biomass for use in preventing or reducing the adverse effects of pathogens in animal or human digestive tract, characterized in that said hydrothermally processed microbial biomass is obtainable by cultivating microorganism on a cultivation medium and subsequently subjecting microbial biomass from said cultivation to a hydrothermal treatment at a temperature of at least 160 °C for 1s to 360 minutes at a pressure above 5 bar.

Yet an aspect relates to a feed or food composition for use in preventing or reducing the adverse effects of pathogens in animal or human digestive tract, characterized in that it comprises the microbial biomass according to the method of the present invention.

**Brief description of the Figures**

Figure 1 shows the binding of microbial biomasses of *Escherichia coli.*

Figure 2 shows the binding of microbial biomasses of *Salmonella enterica.*

Figure 3 shows the binding of microbial biomasses of lysine

Figure 4 shows the binding of microbial biomasses of threonine

Figure 5 shows the binding of microbial biomasses of tryptophan

Figure 6 shows the binding of microbial biomasses of methionine

Figure 7 shows the binding of microbial biomasses of thiamine

Figure 8 shows the binding of microbial biomasses of niacin

**Detailed description of the invention**

**Definitions**

The present invention provides microorganism biomass for use in preventing or reducing the adverse effects of pathogens in animal or human digestive tract. Such biomass is obtainable by cultivating said microorganisms on a cultivation medium, optionally comprising lignocellulosic material, and processing the microbial biomass by hydrothermal processing.

According to this disclosure microbial biomass obtainable by cultivating microorganism strains in a cultivation medium comprising lignocellulosic material, typically lignocellulose hydrolysate or saccharides has been shown to inhibit the binding of pathogens originating from contaminated feed.

By "lignocellulosic material" is meant any material which comprises lignocellulose. Lignocellulosic material comprises lignocellulose, fragments of lignocellulose or hydrolysis products of lignocellulose including saccharides, such as mono-, di-, and/or oligosaccharides originating from lignocellulose. "Lignocellulosic material" may comprise also other components in addition to lignocellulose such as other components from wood or herbaceous plants.

"Lignocellulose" comprises carbohydrate polymers cellulose and hemicellulose and aromatic polymer lignin.Lignocellulosic material include but is not limited to woody plants or non-woody, herbaceous plants or other materials containing cellulose and/or hemicellulose: Materials can be agricultural residues (such as wheat straw, rice straw, chaff, hulls, corn stover, sugarcane, bagasse), dedicated energy crops (such as switchgrass, *Miscanthus,* reed canary grass, willow, water hyacinth), wood materials or residues (including sawmill and pulp and/or paper mill residues or fractions, such as hemicellulose, spent sulphite liquor, waste fibre and/or primary sludge), moss or peat, or municipal paper waste. The term lignocellulosic material comprises also low lignin materials, materials such as macroalgae biomass. In addition, the materials comprise also hemicellulose or cellulose fractions from industrial practises. The term lignocellulosic material encompasses any kind of cellulose fraction. The raw materials or certain fractions, such as hemicellulose and/or cellulose, of raw materials from different origin, plant species, or industrial processes can be mixed together and used as raw materials for cultivating microbial biomass according to this disclosure.

The term lignocellulosic material comprises at least 50 wt% lignocellulose, preferably at least 60 wt% lignocellulose, more preferably at least 70 wt% lignocellulose, most preferably at least 80 wt% lignocellulose. Usually lignocellulosic material comprises 60-95 wt% lignocellulose, typically 70-90 wt% or 80-90 wt% lignocellulose.

"A cultivation medium comprising lignocellulosic material" means a cultivation medium for cultivating a microorganism, which medium comprises lignocellulose, fragments of lignocellulose or hydrolysis products of lignocellulose including, nitrogen compounds originating from proteins and metals, and other components necessary for cultivating said microorganism, such as a source of nitrogen, phosphorus, inorganic salts and/or trace elements. Lignocellulose may function as a carbon source for said microorganism, but it may also have other functions in the cultivation medium.

"Hydrolysis" refers here to saccharification of polymeric sugars to sugar oligomers and monomers. Saccharification is typically carried out in two phases: first the substrate i.e. lignocellulosic material or lignocellulose is hydrolyzed by thermochemical or chemical methods and then by using enzymes capable of hydrolysing polymeric sugars. Alternatively and depending on the lignocellulosic material saccharification can be carried out by using thermochemical or chemical methods or by enzymes capable of hydrolysing polymeric sugars or some combination of these methods. Chemical methods include, but are not limited to acid treatment.

In some embodiments of the invention, the microorganism cultivated on a medium comprising lignocellulosic material is able to hydrolyse lignocellulose to sugar oligomers and monomers. In other preferred embodiments lignocellulosic material or feedstock comprising polymeric sugars is hydrolyzed to monomeric sugars thermochemically and/or chemically and/or by enzymes before cultivation. In preferred embodiments of the invention, lignocellulosic material containing polymeric sugars is hydrolysed thermochemically and/or chemically and/or by enzymes to contain oligomeric sugars and the microorganism cultivated on a medium comprising lignocellulose is able to utilize these sugar oligomers.

By "lignocellulose hydrolysate" is meant the hydrolysis products of lignocellulose or lignocellulosic material comprising cellulose and/or hemicellulose, oligosaccharides, mono- and/or disaccharides, acetic acid, formic acid, other organic acids, furfural, hydroxymethyl furfural, levulinic acid, phenolic compounds, other hydrolysis and/or degradation products formed from lignin, cellulose, hemicellulose and/or other components of lignocellulose, nitrogen compounds originating from proteins, metals and/or non-hydrolyzed or partly hydrolyzed fragments of lignocellulose.

Hydrothermal processing or hydrothermal treatment or heat treatment are used here synonymously and refer to a process in which an aqueous suspension comprising microbial biomass is heated to a temperature of at least 160°Cand subjecting the aqueous suspension comprising said microbial biomass to a temperature of at least 160°C for 1s to 360 minutes at a pressure above 5 bar.

It should be notes that in the context of the present invention the above mentioned methods, i.e. thermochemical treatment, steam explosion, hot water extraction, autohydrolysis, sub critical water treatment, super critical water treatment, strong acid treatment, mild acid treatment, alkaline treatment (e.g. lime, ammonia), Organosolv treatment (e.g.

alcohols, organic acids), mechanical treatment, thermomechanical treatment and ionic liquid treatment does not fall within the definition of hydrothermal processing (or hydrothermal treatment, heat treatment, HTT).

The term "severity factor" (Pedersen, M. and A. S. Meyer (2010). Lignocellulose pretreatment severity - relating pH to biomatrix opening. New Biotechnology 27(6): 739-750) refers here to a parameter $LogR_0$, which is calculated based on the following equation and which describes the hydrothermal conditions in terms of temperature and time.

$$R_0 = \int_a^b \exp\left(\frac{T(t) - 100}{14.75}\right) dt = t \cdot \exp\left(\frac{T(t) - 100}{14.75}\right)$$

According to this disclosure the treatment of lignocellulose and production of lignocellulose hydrolysate for cultivation of microbial biomass can be done with any method known in the art or developed in the future. Methods include but are not limited to thermochemical treatment, steam explosion, hot water extraction, autohydrolysis, sub critical water treatment, super critical water treatment, strong acid treatment, mild acid treatment, alkaline treatment (e.g. lime, ammonia), Organosolv treatment (e.g. alcohols, organic acids), mechanical treatment, thermomechanical treatment, and ionic liquid treatment. These treatments methods can be combined with enzymatic treatment.

"A cultivation medium comprising saccharides" means here a cultivation medium, which comprises mono-, di-, and/or oligosaccharides from lignocellulose.

"A cultivation medium comprising pure saccharides" means here a cultivation medium, which comprises mono-, di- and/or oligosaccharides, which are not produced by hydrolysis of lignocellulose. Pure saccharides include, e.g. starch or starch derived sugars, sugar cane or sugar beet derived sugars.

In some embodiments of the invention, a microorganism is preferably cultivated on a medium comprising lignocellulose hydrolysate, in other embodiments preferably on a medium comprising mono-, di-, and/or oligosaccharides originating from lignocellulose or on pure saccharides. Depending on the pathogen either the whole range of hydrolysis products of lignocellulose, or saccharides (mono-, di- and/or oligosaccharides) obtained as hydrolysis products from lignocellulose or pure saccharides, give more effective biomass for preventing or reducing the adverse effects of said pathogen.

By "a microorganism" is meant any microorganism, such as a fungus, preferably a filamentous fungus or yeast, an alga including heterotrophic alga, a bacterium or an archaebacterium, which microorganism is capable of producing cellular biomass. Microorganism is able to produce microbial biomass when grown on cultivation medium comprising a source of carbon. Preferably said microorganism is a filamentous fungus or yeast. Most preferably the biomass is obtained from filamentous fungi from genus *Aspergillus* and/or *Mortierella* or yeasts from genus *Rhodosporidium, Rhodotorula, Cryptococcus, Lipomyces* or *Saccharomyces.*

In the context of the present invention the term "biomass" (or "cell mass") refers to biological material derived from living or non-living organisms. "Microbial biomass" or "microorganism biomass" thus refers to biomass derived from a microorganism(s). The microbial biomass may be obtained from a single species of microorganism or a pool of different species of microorganisms. The microbial biomass may exist in a living or a non-living state. In the context of the present invention, the microbial biomass, which has been subjected to hydrothermal heat treatment (HTT) is in a non-living state.

"Hydrothermally processed microbial biomass" thus refers to microbial biomass, which have been subjected to hydrothermal processing. It follows from the above that hydrothermally processed microbial biomass according to this disclosure refers to biomass of microorganisms in a non-living state since the hydrothermal processing of microbial biomass disrupts the structure of the biomass cells.

In the context of the present invention the term "residual microbial biomass" refers to microbial biomass in the form of a by-product from a process that actively involves or is conducted in the present of microorganism. For example the residual microbial biomass may be the microorganisms from a fermentation broth such as an alcohol fermentation broth or microbial lipid production process. The residual microbial biomass may be in a living state or an non-living state. It follows that if the process from which the residual microbial has been subjected to a step that terminates the fermentation by killing the microbes present (e.g. pasteurization) then the residual microbial biomass is in a non-living state. According to this disclosure a microorganism can be cultivated for biomass production or it can be cultivated under conditions that permit the microorganism to produce desired products, such as lipids, or other economically valuable co-products. Typically the microbial biomass according to this disclosure is obtained after recovery of lipids from microbial biomass.

By "lipid producing microorganism " or "oleaginous microorganism" is meant a microorganism that is able to produce and accumulate in their cell biomass more than 15% lipids from their dry cell biomass weight when cultivated in suitable conditions for lipid production. Alternatively or in addition microorganism may be able to excrete lipids outside the cells e.g. to cultivation medium.

Single-cell oil stands typically for an intracellular lipid that has been intracellularly synthesized by a microorganism, lipids excreted by the cell, as well as lipids present in the structural parts of a cell, such as in membrane systems.

By "single-cell oil production process" is meant a process where microorganisms are used to produce oils. In the process, microorganisms are cultivated on organic carbon sources and microorganisms are let to produce oil. Microorganisms can store the oil intracellularly or excrete it out from the cell. Organic carbon source can be pure saccharides, lignocellulosic material, or starch. Single-cell oil process typically utilizes microorganisms, such as oleaginous microorganisms, that are capable of producing lipids efficiently.

"Lipid recovery" or" oil recovery" refers to a process, in which the lipid (intracellular lipid) is recovered by mechanical, chemical, biochemical, thermomechanical or autocatalytic methods or by a combination of these methods from the microorganism cells.

After cultivation of microorganisms for production of oil, microorganisms containing lipids may be separated from culture medium by any known methods, such as by using a filtration or decanting techniques. Alternatively, centrifugation with industrial scale commercial centrifuges of large volume capacity may be used to separate the desired products.

In various embodiments of the invention, oil, or precursors for oil, may be recovered from cell biomass for example cell biomass provided in the form of a culture broth using any method known in the art or developed in the future. Such methods, include, but are not limited to extraction with organic solvents or mechanical pressing. The microbial biomass is subjected to hydrothermal processing before or after the recovery of oil or precursors of oil from the cell biomass. Preferably the microbial biomass is hydrothermally processed before the recovery of oil or oil precursors.

In embodiments where microbial biomass is cultivated on lignocellulosic material, in particular lignocellulose hydrolysate, it may comprise also hydrolysis products or residues of lignocellulose. Such residues are for example, acetic acid, formic acid, other organic acids, furfural, hydroxymethyl furfural, levulinic acid, phenolic compounds, other hydrolysis or degradation products formed from lignin, cellulose or hemicellulose or other components of lignocellulose, or non-hydrolyzed or partly hydrolyzed fragments of lignocellulose. Hence, if microbial biomass is cultivated on lignocellulose hydrolysate, it typically comprises lignocellulose hydrolysis products or residues, which the microorganism has not utilized during cultivation or which the microorganism has not been able to utilize, for example lignin, polysaccharides and mono-, di- or oligosaccharides. The amount of lignocellulose hydrolysis products or residues is typically 0.01 - 20 wt% (dry weight), usually 0.5 - 10 wt % of dry weight of the microbial biomass. The amount of phenolic compounds originating from lignocellulosic materials is typically 0.01 - 10 wt% (dry weight), usually 0.05 - 5 wt % of dry weight of the microbial biomass. Depending on the source of the lignocellulosic material and on other components of the cultivation medium, "microbial biomass" may comprise for example residues of various proteins and lipids.

In one embodiments of the invention, the microorganism is cultivated on lignocellulosic hydrolysate containing lignocellulose hydrolysis or degradation products not utilized by microorganism, such as phenolic compounds and furfural. These compounds are formed in the hydrolysis of lignocellulose. The lignocellulose hydrolysis can be performed by thermochemical treatment, steam explosion, hot water extraction, autohydrolysis, sub critical water treatment, super critical water treatment, strong acid treatment, mild acid treatment, alkaline treatment (e.g. lime, ammonia), Organosolv treatment (e.g. alcohols, organic acids), mechanical treatment, thermomechanical treatment, ionic liquid treatment in addition to enzymatic treatment. Without being bound by any theory it is also considered that lignocellulosic residues if present in the hydrothermally processed microbial biomass may be at least partly responsible for the prevention or adverse effect of pathogens in digestive tract. The components of hydrolysate, such as phenolic compounds and metals may adsorb to the cell wall of microorganisms and be carried on with the microbial biomass. It is also considered that at least part of the lignocellulosic material dissolved in the cultivation medium is transformed into solid state during the hydrothermal processing and therefore to form part of the microbial biomass. Alternatively the microbial cell walls may be modified by the components of lignocellulose hydrolysate. The capability of hydrothermally processed microbial biomass to prevent or to reduce the adverse effect of pathogens is considered to be due to process driven changes in cell structure of the biomass and/or changes in the cell wall composition of the biomass. These changes in biomass cell structure and composition of the cell wall and the biomass are considered to be due to the hydrothermal processing.

Preferred microorganism strains for the purposes of the present invention are from the species and genera listed below:

Preferred fungal strains are from species from genera *Aspergillus* such as Aspergillus oryzae, *Mortierella such as Mortierella isabellina, Chaetomium, Claviceps, Cladosporidium, Cunninghamella, Emericella, Fusarium, Glomus, Mucor, Paecilomyces, Penicillium, Pseudozyma, Pythium, Rhizopus, Tremella,Trichoderma, Zygorhynchus, Humicola, Cladosporium, Malbranchea, Umbelopsis such as Umbelopsis isabellina and Ustilago*

Preferred yeast strains are those belonging to species from genera *Clavispora, Geotrichum, Deparyomyces, Pachysolen, Kluyveromyces, Galactomyces, Hansenula, Leucosporidium, Saccharomyces, Sporobolomyces, Sporidiobolus, Waltomyces, Endomycopsis, Cryptococcus,* such as *Cryptococcus curvatus, Rhodosporidium,* such as *Rhodosporidium toruloides or Rhodosporidium fluviale, Rhodotorula,* such as *Rhodotorula glutinis, Yarrowia,* such as *Yarrowia lipolytica, Pichia,* such as *Pichia stipitis, Candida* such as *Candida curvata, Lipomyces* such as *Lipomyces starkeyi* and *Trichosporon* such as *Trichosporon cutaneum* or *Trichosporon pullulans.* Most preferred yeasts are from genus *Rhodosporidium, Rhodotorula, Cryptococcus, Lipomyces* or *Saccharomyces.*

Preferred bacteria are those belonging to the species from genera *Acinetobacter, Actinobacter, Alcanivorax, Aerogenes, Anabaena, Arthrobacter, Bacillus, Clostridium, Dietzia, Gordonia, Escherichia, Flexibacterium, Micrococcus, Mycobacterium, Nocardia, Nostoc, Oscillatoria, Pseudomonas, Rhodococcus, Rhodomicrobium, Rhodopseudomonas, Shewanella, Shigella, Streptomyces* and *Vibrio.*

Most preferred algae are microalgae, such as microalgae species from genera *comprising Achnantes, Amphiprora, Amphora, Ankistrodesmus, Attheya, Boeklovia, Botryococcus, Biddulphia, Brachiomonas, Bracteococcus, Carteria, Chaetoceros, Characium, Chlamydomonas, Crypthecodinium, Cryptomonas, Chlorella, Chlorococcum, Chrysophaera, Coccochloris, Cocconeis, Cyclotella, Cylindrotheca, Dunaliella, Ellipsoidon, Entomoneis, Euglena, Eremosphaera, Extubocellulus, Franceia, Fragilaria, Gleothamnion, Hantzschia, Haematococcus, Hormotilopsis, Hymenomonas, Isochrysis, Lepocinclis, Melosira, Minidiscus, Micractinum, Monallanthus, Monoraphidium, Muriellopsis, Nannochloris, Nannochloropsis, Navicula, Neochloris, Nephroselmis, Nitzschia Ochromonas, Oedogonium, Oocystis, Papiliocellulus, Parachlorella, Pascheria, Pavlova, Peridinium, Phaeodactylum, Plankthothrix, Platymonas, Pleurochrysis, Pleurosigma, Porphyridium, Prototheca, Prymnesium, Pseudochlorella, Pyramimonas, Pyrobotrus, Radiosphaera, Rhodomonas, Rhodosorus, Sarcinoid, Scenedesmus, Schizochytrium, Scrippsiella, Seminavis, Skeletonema, Spirogyra, Stichococcus, Synedra, Tetraedron, Tetraselmis, Thalassiosira, Trachyneis, Traustrochytrium, Trentepholia, Ulkenia, Viridiella, and Volvox.* The organisms belonging to the genera *Schizochytrium, Thraustochytrium* and Crypthecodinium and Ulkenia are sometimes called as marine fungi.

According to the present disclosure hydrothermally processed microbial biomass obtainable by cultivating microorganisms on a cultivation medium prevents or reduces the adverse effects of pathogens in animal or human digestive tract, preferably in animal or human stomach, small intestine and/or colon.

Preferably said biomass prevents or reduces the adverse effects of pathogens. By "pathogen" is hear meant any microorganism, typically a bacterium or a fungus that causes disease or disorder in animal or human.

Pathogens comprise species for example from genera *Salmonella, Shigella, Campylobacter, Clostridium,* e.g. *C. difficile* or C. perfringens , *Vibrio,* e.g. V. *cholera, V. parahaemolyticus, Staphylococcus aureus* and *Escherichia coli* . Preferably said biomass prevents or reduces the adverse effects of pathogens *Escherichia coli* and/or *Salmonella enterica.*

By "preventing or reducing the adverse effects of pathogens" is meant here the increase in well-being and productivity of animals, the productivity of said animals being decreased by animal diseases or disorders caused by pathogens, or high mortality of said animals being caused by pathogens. In humans preventing or reducing the adverse effects of pathogens means the increase in well-being and better health.

The present invention provides also a feed or food composition for use in preventing or reducing the adverse effects of pathogens in animal or human digestive tract. Said feed or food composition comprises the hydrothermally processed microbial biomass according to this disclosure.

The use of a feed composition for use in reducing the amount of pathogens in feed results also in reduction of pathogens in products sold to end consumers, such as milk, eggs, or meat.

The feed or food composition comprises hydrothermally processed microbial biomass preferably 0.001-20 wt %, more preferably 0.1-3 wt % dry weight of dry weight of said composition.

In some embodiments of the invention the composition is a ruminant or monogastric animal, such as pigs or poultry feed composition. The composition comprises hydrothermally processed microbial biomass and components or ingredients suitable for ruminant or monogastric animal feed composition, such as source of protein and fibre components.

In some other embodiments of the invention the composition is an aquaculture such as fish, mollusc, or shrimp feed composition. The composition comprises microbial biomass and components or ingredients suitable for fish feed composition, such as source of protein and fat components.

In further embodiments of the invention the food composition is a nutritional product for human use. The composition comprises microbial biomass and components or ingredients suitable for food composition. Such components comprise for example salt and sugar or other flavour giving ingredients.

By "feed or food ingredient" is here meant a component part or constituent of any combination or mixture making up a feed or food, whether or not it has a nutritional value in the animal's or human's diet. Ingredients are of plant, animal or aquatic origin, or other organic or inorganic substances.

In the context of the present invention "feed or food additive" refers to a composition of microbial biomass, which essentially does not have a nutritional value in the animal's or human's diet. The "feed or food additive" of the present invention has the capacity of inactivating a pathogen.

The feed or food composition for use in preventing or reducing the adverse effects of pathogens in animal or human digestive tract may be used in the composition as a food or feed topping, food or feed extender or feed or food supplement.

The present invention provides also a feed or food composition, which comprises preferably 0.001-20 wt %, more preferably 0.1-3 wt % dry weight of dry weight of said composition non-living microbial biomass, said microbial biomass being obtainable as disclosed herein, and preventing or reducing the adverse effects of pathogens in animal or human digestive tract.

By the term "dry matter content" or "DM" is meant the solid contents of the suspension that are being subjected to hydrothermal treatment. According to the invention the DM of the suspensions subjected to hydrothermal treatment is in the range of about 1% to about 25% such as e.g. 5% to about 20%, such as e.g. about 8 % to about 20 % and can thus be characterized as clar suspensions. It should be noted that the suspension according to the invention has a maximum DM content of 25%.

Also described is a method for producing a feed or food additive. In broad terms the method comprises the steps of

- providing an aqueous suspension comprising microbial biomass,

- subjecting the microbial biomass to a hydrothermal processing at a temperature of at least 160 °C

- separating a solid phase containing microbial biomass and/or separating the cell wall from the soluble intracellular components and obtaining a cell wall extract,

- optionally, drying the separated microbial biomass or cell wall extract,

- subsequently said cell biomass or cell extract may be added to a feed or food product.

Described is a method for preparing a feed or food additive, said method comprising the steps of

(a) providing an aqueous suspension comprising microbial biomass,

(b) subjecting said aqueous suspension comprising said microbial biomass to a temperature of at least 160°C for 1s to 360 minutes at a pressure above 5 bar,

(c) separating a solid phase containing the microbial biomass,

(d) optionally, drying the microbial biomass obtained from step (c).

The microbial biomass obtained in step (c) may be in the form of a concentrated suspension comprising said solid phase containing the microbial biomass microbial biomass, a wet or a substantially dry form of said solid phase containing the microbial biomass microbial biomass. Where it is desired, a dry form of the solid phase containing the microbial biomass may be obtained by subjecting the solid phase to a step of drying the solid phase.

The hydrothermal processing (step (b)) is conducted at a pressure above 5 bar, such as in the range of 6 to 25 bar, for example in the range of 10 to 25 bar, such as in the range of 10 to 15 bar. The hydrothermal processing (step (b)) is conducted for 1 second to 360 minutes, such as in the range of 5 minutes to 240 min, for example in the range of 10 minutes to 120 min.

In one embodiment of the present invention, the hydrothermal processing (step (b)) is conducted in the range of 180 °C to 250 °C. In a preferred embodiment, the hydrothermal processing (step (b)) is conducted under conditions corresponding to a severity factor of at least 2.5, preferably of at least 3.5, most preferably at least 3.9.

In a further embodiment, the severity factor is in the range of of about 3.5 to about 5.5 such as e.g. about 4 to about 4.9, such as e.g. about 4.1 to about 4.8. The thermal treatment is typically conducted in a closed vessel.

Moreover, the hydrothermal heating is conducted during a period of e.g. about 5 minutes and up to about 120 minutes, such as e.g. about 10 minutes and up to about 60 minutes, while the temperature is about 160°C and up to about 220°C, such as e.g. about 180°C and up to about 220°C.

In another embodiment, the microbial biomass obtained from (c) is subjected to washing step.

Where the microbe has been cultivated using a cultivation medium comprising lignocellulose the cultivation medium may comprise lignocellulose hydrolysate if not before then at least after the hydrothermal treatment (step (b)).

In one embodiment of the present invention, the aqueous suspension comprising microbial biomass contains cultivation medium comprising lignocellulose hydrolysate. In a further embodiment, wherein the microbial biomass obtained in step (c) further comprises residues of lignocellulose.

The amount of lignocellulose hydrolysis products or residues is typically 0.01 - 20 wt% (dry weight), usually 0.5 - 10 wt % of dry weight of the microbial biomass. The amount of phenolic compounds originating from lignocellulosic materials is typically 0.01 - 10 wt% (dry weight), usually 0.05 - 5 wt % of dry weight of the microbial biomass.

In another embodiment, the aqueous suspension comprising microbial biomass contains cultivation medium comprising starch and/or sugar cane/beet derived sugars.

According to this disclosure the microbial biomass obtainable by cultivating microorganisms on a medium comprising pure saccharides, lignocellulosic material or starch is useful in preventing or reducing the adverse effects of pathogens in animal or human digestive tract.

In a preferred embodiment of the present invention, the microbial biomass is obtained from oleaginous microorganisms.

It follows that the feed or food additive of the present invention may in the form of a by-product from a microbial lipid production process. The by-product may be subjected to further refining to obtain the feed or food additive.

Accordingly, in one embodiment, said microbial biomass is residual microbial biomass obtained from a fermentation process. In a further embodiment, the microbial biomass of step (c) is obtained by subjecting the oleaginous microorganisms to a step of removing the microbial oil from said microbial biomass. In a particular embodiment, the microbial biomass of step (c) is obtained by subjecting the oleaginous microorganisms to a step of removing the microbial oil from said microbial biomass after step b).

Thus one embodiment of the present invention provides a method for preparing a feed or food additive, said method comprising the steps of

(a) providing an aqueous suspension comprising microbial biomass from or in the form of a oleaginous microorganisms,

(b) subjecting said aqueous suspension comprising said microbial biomass to a temperature of at least 160°C for 1s to 360 minutes at a pressure above 5 bar,

(c) separating a solid phase containing the microbial biomass from the liquid phase,

(d) drying the solid phase containing the microbial biomass obtained from step (c),

(e) extracting the microbial oil from the microbial biomass obtained from step (d) with a solvent,

(f) optionally, drying the microbial biomass obtained from step (e).

The aqueous suspension comprising microbial biomass may contain one or more microorganism or microbial biomass from more than one microorganism. Thus in one embodiment, the microbial biomass is obtained from one or more microorganism. It follows that the feed or food additive of the present invention may contain microbial biomass from more than one microorganism.

As mentioned herein, the microbial biomass used by the invention may be obtained from a wide range of microorganisms including both unicellular and multicellular microorganisms. In a preferred embodiment, the microbial biomass is obtained from one or more fungi, preferably yeasts or filamentous fungi. In one embodiment of the invention the microbial biomass in aqueous suspension used by the method of the invention is obtained from *Rhodosporidium* or *Lipomyces.* In a preferred embodiment the microbial biomass is obtained from or at least a major part of the microbial biomass is obtained from *Rhodosporidium.* In a preferred embodiment, the microbial biomass is obtained from strains *Aspergillus* or *Mortierella.*

The inventors have discovered that the product of the method has the capacity to inactivate microbial pathogens. As mentioned herein, this capacity of the microbial biomass obtained by the method of the invention is considered to be due to the hydrothermal processing (step b)). The microbial biomass obtained by the method of the invention may therefore be used a food or feed additive due to its capacity to prevent or to reduce the adverse effect of a microbial pathogen in the digestive tract of a human or animal subject. The hydrothermal treatment significantly lowers the nutritional value of the microbial biomass and it cannot therefore be used as animal feed or fodder.

The microbial biomass is believed to physically bind pathogen and prevent or reduce the infection in the digestive tract of animals and the subsequent distribution of the blood and target organs.

One aspect of the present invention therefore relates to a method for inactivating a pathogen in a contaminated feed or food product, said method comprising the steps of

(a) providing a feed or food product contaminated with a pathogen (or suspected to be contaminated with a pathogen),

(b) providing a feed or food additive according to the invention,

(c) mixing the feed or food product of (a) with the feed or food additive of (b).

Thus it follows that one embodiment provides a method for inactivating a pathogen contaminated feed or food product, said method comprising the steps of

(a) providing a feed product contaminated with a pathogen (or suspected to be contaminated with a pathogen),
(b) providing a feed additive according to the invention,
(c) mixing the feed product of (a) with the feed additive of (b).

Further described is a method for preparing a feed or food product, said method comprising the steps of

(a) providing a feed or food substance,
(b) providing a feed or food additive according to the invention,
(c) mixing the feed or food substance of (a) with the feed or food additive of (b) to obtain a feed or food product.

In one embodiment, the feed additive used by the above methods amounts 0.001-20 wt % dry weight of the feed of step (c). In another embodiment, the feed additive amounts 0.1-3 wt % dry weight of the feed of step (c).

The feed substance provided in step (a) of the above methods is contaminated with one or more pathogens or at least suspected to be contaminated with one or more pathogens. As mentioned herein, the term "pathogen" means a pathogenic microarganism, typically a bacteria or a fungus. In one embodiment of the present invention the pathogen is selected from the list consisting of *Salmonella* genera and *E. coli* or a combination of said pathogens. In a particular embodiment, the feed or food product is contaminated with a pathogen selected from the list consisting of *Escherichia coli* and *Salmonella enterica* or a combination of said pathogens.

As mentioned above the addition of the feed additive of the present invention reduced the risk of adverse effects of pathogens present in a pathogen contaminated feed product. The additive may thus be added to the feed for a prevention purpose or added to a feed know to be contaminated with one or more pathogens.

One aspect of the present invention provided a method for feeding an animal, said method comprising feeding said animal with a feed product of the present invention.

In one embodiment, said animal is a ruminant or monogastric animal. In another embodiment, said animal is aquaculture such as fish, mollusk or shrimp feed composition. The feed product thus comprises hydrothermally processed microbial biomass and components or ingredients suitable for aquaculture feed composition, such as source of protein and fat components.

In a further embodiment, said animal is a livestock such as a livestock selected from the list consisting of pigs, horses, poultry, cattle, goats and sheep. In a preferred embodiment, the animal is a chicken. In another preferred embodiment, the animal is a pig.

In further embodiments of the invention the food product is a nutritional product for human use. The food product then comprises hydrothermally processed microbial biomass and components or ingredients suitable for food composition. Such components or ingredients comprise for example salt and sugar or other flavour giving ingredients.

The hydrothermally processed microbial biomass may be used in the feed or food product as a food or feed topping, food or feed extender or feed or food supplement.

The present invention uses also in its methods a feed or food composition, which comprises preferably 0.001-20 wt %, more preferably 0.1-3 wt % dry weight of dry weight of said hydrothermally processed microbial biomass, said hydrothermally processed microbial biomass being obtainable as disclosed herein, and preventing or reducing the adverse effects of pathogens in animal or human digestive tract.

Also described is a method for producing a feed or food composition from single cell oil production process. Preferable the method comprises the steps of

- cultivating a microorganism on a cultivation medium;
- allowing microorganisms to produce oil, preferably under nutrient starvation;
- subjecting the microbial biomass to a hydrothermal processing at a temperature of at least 160 °C
- collecting oil-rich microorganism cells from cultivation medium and optionally washing and drying the cells;
- recovering oil from microorganisms cells typically by organic solvent, said extraction creating liquid phase containing oil and residual microbial biomass,
- separating the residual microbial biomass;
- drying and desolventizing the separated residual microbial biomass;
- adding said microbial biomass to feed or food.

Separated residual microbial biomass may optionally be treated mechanically, thermochemically, chemically or enzymatically prior to adding to feed or food.

Advantageously a feed or food composition is prepared by mixing a specific amount of hydrothermally processed microbial biomass or cell wall extract to feed or food. A feed or food composition is prepared to comprise preferably 0.001-20 wt %, more preferably 0.1-3 wt % (dry weight) of dry weight of said composition microbial biomass obtainable as disclosed herein.

Advantageously the hydrothermally processed microbial biomass may comprise residues of lignocellulose. The amount of lignocellulose hydrolysis products or residues is typically 0.01 - 20 wt% (dry weight), usually 0.5 - 10 wt % of dry weight of the microbial biomass. The amount of phenolic compounds originating from lignocellulosic materials is typically 0.01 - 10 wt% (dry weight), usually 0.05 - 5 wt % of dry weight of the microbial biomass.

Further described is a method for improving the well-being and increasing the productivity of an animal, characterized in that said method comprises feeding to said animal a feed composition comprising hydrothermally processed microbial biomass as described herein (the feed additive of the invention). In a preferred embodiment, the composition comprises 0.001-20 wt %, preferably 0.1-3 wt % dry weight of dry weight of said microbial biomass.

A further aspect of the present invention provides a hydrothermally processed microbial biomass for use in preventing or reducing the adverse effects of pathogens in animal or human digestive tract, characterized in that said hydrothermally processed microbial biomass is obtainable by cultivating microorganism on a cultivation medium and subsequently subjecting microbial biomass from said cultivation to a hydrothermal treatment at a temperature of at least 160 °C.

In one embodiment, the hydrothermal treatment is conducted at a temperature of from 180 °C to 250 °C. In another embodiment, the hydrothermal treatment is conducted in conditions corresponding to a severity factor of at least 2.5, preferably of at least 3.5, most preferably at least 3.9. In a further embodiment, the microbial biomass is subjected to washing step after the hydrothermal treatment.

In a further embodiment, the severity factor is in the range of of about 3.5 to about 5.5 such as e.g. about 4 to about 4.9, such as e.g. about 4.1 to about 4.8. The thermal treatment is typically conducted in a closed vessel.

Moreover, the hydrothermal heating is conducted during a period of e.g. about 5 minutes and up to about 120 minutes, such as e.g. about 10 minutes and up to about 60 minutes, while the temperature is about 160°C and up to about 220°C, such as e.g. about 180°C and up to about 220°C.

In yet another embodiment, the cultivation medium comprises lignocellulose hydrolysate. In a particular embodiment, the microbial biomass comprises residues of lignocellulose.

In one embodiment of the present invention, the cultivation medium comprises starch and/or sugar cane/beet derived sugars.

In a preferred embodiment of the present invention the biomass for use in preventing or reducing the adverse effects of pathogens in animal or human digestive tract is residual microbial biomass obtained from a single cell oil production process. In a further embodiment, said biomass is obtainable by subjecting the microbial biomass to hydrothermal treatment before recovering the single cell oil from said microbial biomass.

In another embodiment, the microbial biomass is residual microbial biomass obtained from an alcohol production process, preferably from an ethanol production process.

As mentioned herein the microbial biomass is obtainable by cultivating fungi strains, preferably yeasts or filamentous fungi. In a preferred embodiment, the microbial biomass is obtained from strains of yeasts belonging to genus *Rhodosporidium, Rhodotorula, Cryptococcus, Lipomyces,* or *Saccharomyces*. In a preferred embodiment, the microbial biomass is obtained from strains As*pergillus* or *Mortierella.*

According to the present invention the microbial biomass prevents or reduces the adverse effects of one or more pathogens in animal or human stomach, small intestine and/or colon.

The present invention further provide a feed or food composition (also referred to as a feed or food product) for use in preventing or reducing the adverse effects of pathogens in animal or human digestive tract, characterized in that it comprises the hydrothermally processed microbial biomass described herein (also referred to a feed or food additive).

In one embodiment, the composition comprises the microbial biomass 0.001-20 wt %, preferably 0.1-3 wt % dry weight of dry weight of said composition. In a further embodiment, the composition comprises 0.001-20 wt % dry weight of dry weight of said composition microbial biomass.

As discussed herein the feed composition comprising the hydrothermally processed biomass is useful for preventing or reducing the adverse effects of pathogens in animal. Animals include a ruminant or monogastric animal. Thus in one embodiment of the present invention, the feed composition is a ruminant or monogastric animal feed composition. Animals also include an aquatic organisms, such as fish, mollusk or shrimp feed. Thus in yet another embodiment, the composition is an aquaculture feed composition such as fish, mollusk or shrimp feed composition.

In a further embodiment of the invention the food composition is a nutritional product for human use. In a particular embodiment, the hydrothermally processed microbial biomass is used in the composition as a food or feed topping, food or feed extender or food or feed supplement.

Advantageously a feed or food composition is prepared by mixing a specific amount of microbial biomass or cell wall extract to feed or food. A feed or food composition is prepared to comprise preferably 0.001-20 wt %, more preferably 0.1-3 wt % (dry weight) of dry weight of said composition microbial biomass obtainable as disclosed herein.

The present invention provides also a method for improving the well-being and increasing the productivity of an animal. The method comprises feeding to said animal a feed composition comprising non-living microbial biomass, said biomass being obtainable by cultivating a microorganism on a cultivation medium comprising lignocellulosic material as disclosed herein.

Preferably the composition comprises 0.001-20 wt %, more preferably 0.1-3 wt % dry weight of dry weight of said composition microbial biomass.

According to this disclosure the microbial biomass obtainable by cultivating microorganisms on a medium comprising pure saccharides or lignocellulosic material is used to prevent or reduce the adverse effects of pathogens in animal or human digestive tract. The prevention or reduction effect is proposed to be based on the ability of the biomass to inhibit the adherence of pathogens in animal or human digestive tract and thereby prevent pathogens from entering the rest of the body. Inhibition of pathogens binding to the intestinal epithelium can be due to efficient co-flocculation of the pathogenic bacteria with insoluble part of the microbial biomass, which prevents bacterial binding and leads to washout of the agglomerates. The other possible mechanism is that the microbial biomass masks the adhesins on the surface of the pathogenic bacteria or receptors on the surface of the intestinal epithelium, thus preventing the adherence.

The present invention has been exemplified by showing that hydrothermally processed and oil extracted yeast biomass obtainable from cultivation of *Rhodosporidium* with pure saccharides or with lignocellulosic hydrolysate can be used as an effective binding agent for pathogens in the animal digestive tract. It is also shown that the hydrothermally processed and oil extracted yeast biomasses do not bind essential micronutrients such as vitamins and amino acids.

The hydrothermally processed microbial biomasses from microorganisms grown on pure sugar, or lignocellulosic material or starch inhibit also the binding of other pathogens to intestinal epithelium than those described in the Examples and reduce the adverse effects by other pathogens than those described in the Examples to animals or humans.

Hydrothermally processed and oil extracted yeast biomass originating from cultivation using pure saccharides or lignocellulosic hydrolysate was used to bind pathogens. The ability of hydrothermally processed and oil extracted yeast biomass of genus *Rhodosporidium* to bind *E. coli* and S. *enterica* serovar Dublin was studied and compared with yeast cell wall (YCW), yeast biomass hydrolysate (YH) and with *Rhodosporidium* biomass cultivated using pure saccharides or lignocellulosic hydrolysate and which was not harvested by using hydrothermal processing.

The advantage of the present invention is the enhanced ability of the hydrothermally processed biomass to bind pathogens compared to reference materials YCW, YH, and yeast biomass, which has not been subjected to hydrothermal processing as shown in the experimental part of this disclosure.

## Examples

### Example 1

#### Preparation of hydrothermally processed *Rhodosporidium* biomass grown on pure saccharides for pathogen binding examples

*Rhodosporidium toruloides* strain CBS 8587 (or other R. *toruloides* strain, which are readily available from recognized microbial culture collections) was grown under aeration in a pilot fermentor. Fermentation was done as fed-batch fermentation using glucose as carbon source. After 24 h batch phase glucose syrup was added to the fermentor periodically during the 143 h cultivation. Growth medium was supplemented with yeast extract (8 g/l), $(NH_4)_2SO_4$ (3 g/l), $MgCl_2$ (2 g/l), $K_2HPO_4$ (9 g/l) and $CaCl_2$ (0,4 g/l) and trace minerals $ZnSO_4$ (0,0003 g/l), CuCl (0,0002 g/l) and $MnCl_2$ (0,03 g/l).

After cultivation the biomass was harvested by thermal treatment using two different treatment conditions. Biomass that is referred as Mass 1 was heated in a 1 liter laboratory pressure vessel to 180 C and kept in that temperature for 60 minutes. After the thermal treatment the biomass was separated from the liquid phase by filtration. The biomass was not washed in the filter. The resulting biomass was dried in an oven, milled and extracted using heptane. Residual solvent was removed by drying the biomass using efficient ventilation. This biomass was used in pathogen binding tests and is named Mass 1.

Biomass that is referred as Mass 2 was harvested by heating the biomass containing fermentation broth in a 1 liter laboratory pressure vessel to 210 °C and kept in that temperature for 10 minutes. After the thermal treatment the biomass was separated from the liquid phase by filtration. The biomass not washed in the filter. The resulting biomass was dried in an oven, milled and extracted using heptane. Residual solvent was removed by drying the biomass using efficient ventilation. This biomass was used in pathogen binding tests and is named Mass 2.

Biomass that is referred as Mass 3 was harvested by heating the biomass containing fermentation broth in a 1 liter laboratory pressure vessel to 140 °C and kept in that temperature for 15 minutes. After the thermal treatment the biomass was separated from the liquid phase by filtration. The biomass was not washed in the filter. The resulting biomass was dried in an oven, milled and extracted using heptane. Residual solvent was removed by drying the biomass using efficient ventilation. This biomass was used in pathogen binding tests and is named Mass 3.

**Example 2**

**Preparation of hydrothermally processed *Rhodosporidium* biomass grown on lignocellulosic hydrolysate for pathogen binding examples**

*Rhodosporidium toruloides* strain CBS 8587 (or other *R. toruloides* strain, which are readily available from recognized microbial culture collections) was grown under aeration in a pilot fermentor. Fermentation was done as fed-batch fermentation using lignocellulosic hydrolysate syrup as the carbon source. After 12 h batch fermentation lignocellulose hydrolysate syrup was added to the fermentor periodically during the 144 h cultivation. Growth medium was supplemented with yeast extract (17 g/l), $(NH_4)_2SO_4$ (2,1 g/l), $(NH_4)_2HPO_4$ (2,1 g/l) $MgCl_2$ (3,3 g/l), $K_2HPO_4$ (5 g/l) and $CaCl_2$ (0,2 g/l) and trace minerals $ZnSO_4$ (0,0005 g/l), $CuCl$ (0,0003 g/l) and $MnCl_2$ (0,05 g/l). Lignocellulosic hydrolysate used in the cultivation was prepared by thermo-chemical processing of straw.

After cultivation the biomass was harvested by thermal treatment using three different treatment conditions. Biomass that is referred as Mass 4 was heated in a 1 liter laboratory pressure vessel to 180 °C and kept in that temperature for 60 minutes. After the thermal treatment the biomass was separated from the liquid phase by filtration. The biomass was washed using tap-water in the filter. The resulting biomass was dried in an oven, milled and extracted using heptane. Residual solvent was removed by drying the biomass using efficient ventilation. This biomass was used in pathogen binding tests and is named Mass 4.

Biomass that is referred as Mass 5 was heated in a 1 liter laboratory pressure vessel to 180 °C and kept in that temperature for 60 minutes. After the thermal treatment the biomass was separated from the liquid phase by filtration. The biomass was not washed in the filter. The resulting biomass was dried in an oven, milled and extracted using heptane. Residual solvent was removed by drying the biomass using efficient ventilation. This biomass was used in pathogen binding tests and is named Mass 5.

Biomass that is referred as Mass 6 was heated in a 1 liter laboratory pressure vessel to 220 °C and kept in that temperature for 20 minutes. After the thermal treatment the biomass was separated from the liquid phase by filtration. The biomass was washed using tap-water in the filter. The resulting biomass was dried in an oven, milled and extracted using heptane. Residual solvent was removed by drying the biomass using efficient ventilation. This biomass was used in pathogen binding tests and is named Mass 6.

**Example 3**

**Preparation of *Rhodosporidium* biomass grown on pure saccharides without hydrothermal treatment for pathogen binding examples**

*Rhodosporidium toruloides* strain CBS 8587 (or other *R. toruloides* strain, which are readily available from recognized microbial culture collections) was grown under aeration in a pilot fermentor. Fermentation was done as fed-batch fermentation using glucose as carbon source. After 24 h batch phase glucose syrup was added to the fermentor periodically during the 143 h cultivation. Growth medium was supplemented with yeast extract (8 g/l), $(NH_4)_2SO_4$ (3 g/l), $MgCl_2$ (2 g/l), $K_2HPO_4$ (9 g/l) and $CaCl_2$ (0,4 g/l) and trace minerals $ZnSO_4$ (0,0003 g/l), $CuCl$ (0,0002 g/l) and $MnCl_2$ (0,03 g/l).

After cultivation the biomass was harvested by centrifugation. The biomass was dried in an oven, milled and extracted using heptane. Residual solvent was removed by drying the biomass using efficient ventilation. This biomass was used in pathogen binding tests and is named Mass 7.

**Example 4**

**Preparation of *Rhodosporidium* biomass grown on lignocellulosic hydrolysate without hydrothermal treatment for pathogen binging examples**

*Rhodosporidium toruloides* strain CBS 8587 (or other *R. toruloides* strain, which are readily available from recognized microbial culture collections) was grown under aeration in a 10-liter fermentor. Fermentation was done as fed-batch fermentation using lignocellulosic hydrolysate syrup as the carbon source. After 10 h batch fermentation lignocellulose hydrolysate syrup was added to the fermentor periodically during the 95 h cultivation. Growth medium was supplemented with yeast extract (8 g/l), $(NH_4)_2SO_4$ (2,5 g/l), $MgSO_4$ (2,5 g/l), $KH_2PO_4$ (3,5 g/l), $K_2HPO_4$ (1,5 g/l) and $CaCl_2$ (0,1 g/l) and trace minerals $ZnSO_4$ (0,0008 g/l), $CuCl$ (0,00008 g/l), $MnSO_4$ (0,0008 g/l), $FeSO_4$ (0,0004 g/l)

and NaH$_2$PO$_4$ (0,5 g/l). Lignocellulosic hydrolysate was prepared by thermo-chemical process using straw as a raw material.

After cultivation the biomass was harvested by centrifugation. The biomass was dried in an oven, milled and extracted using heptane. Residual solvent was removed by drying the biomass using efficient ventilation. This biomass was used in pathogen binding tests and is named Mass 8.

**Example 5**

**Pathogen binding tests with the *Rhodosporidium* biomasses prepared in examples 1-4**

Tests to determine the effects of microbial biomasses as inhibitors of intestinal pathogen binding were carried out in the following way:

Microtitre plates were coated with authentic intestinal mucus from piglets. Unbound mucus was washed away with Hepes-Hanks buffer. Test products (test biomasses) were introduced into mucus coated microtiter wells. Radioactively labeled pathogen strains of *E. coli* (F4+) and *S. enterica* (serotype Dublin) in Hepes-Hanks buffer were introduced into the wells. After one-hour incubation at 37 °C loose bacteria were washed off with Hepes-Hanks buffer, scintillation liquid was added and the remaining radioactivity was measured. The remaining radioactivity was proportional to the number of adhered pathogenic bacteria. The number of pathogens bound in the wells with no potential adherence inhibitor (without biomass) (negative control) was compared to those with the test products.

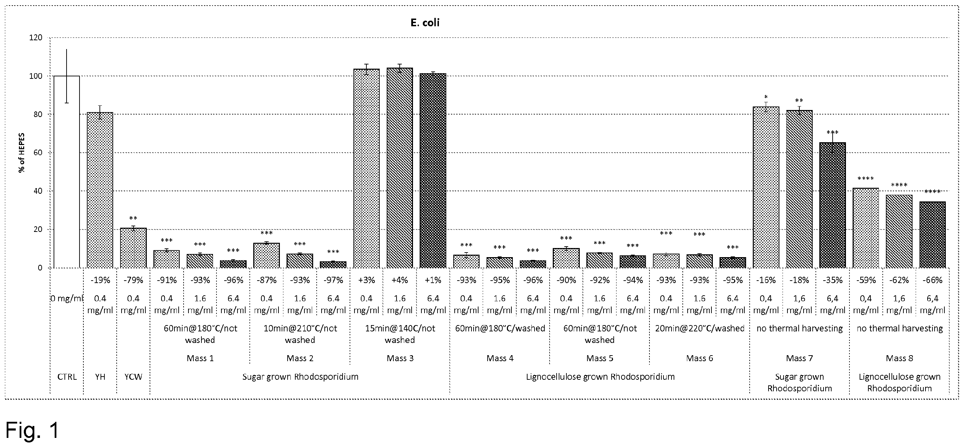

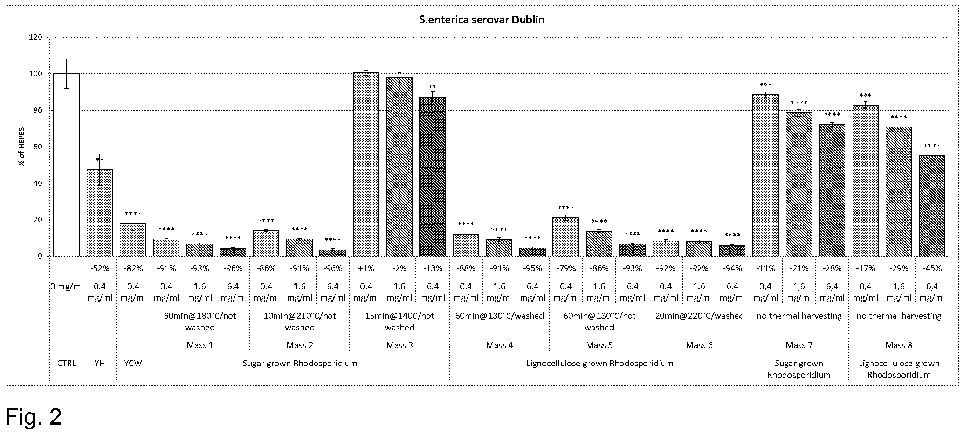

The test products were the biomasses whose production was described in examples 1-4. These biomasses are named as Mass 1, Mass 2, Mass 3, Mass 4, Mass 5, Mass 6, Mass 7 and Mass 8 in Figures 1 and 2.

Yeast biomass hydrolysate was used as positive control at 0.4mg /ml dose (In Fig. 1 and 2 YH).

Also yeast cell wall was used as positive control at 0.4mg /ml dose (In Fig. 1 and 2 YCW).

No amendment was used as a negative control.

**Test treatments**

Three test product doses were used in the model, 0.4, 1.6 and 6.4 mg/ml. Experiments were done by using four replicates.

**Effect of *Rhodosporidium* biomasses on the binding of E. *coli* F4+ (K88) on piglet intestinal epithelium.**

The identities of test products and doses are shown in Figure 1 below the patterns of columns. Hundred % adherence of pathogens is in the absence of any test products (HEPES-Hanks buffer). Error bars indicate SE between 4 replicate reaction vessels and the number in the base of the column indicates the percentage difference when compared to HEPES. The asterisks in Figure 1 shows the statistical significance of the difference to control with no test product according to the Student's t-test (p-value < 0.05 *, p-value < 0.01 **, p-value < 0.001 ***, p-value < 0.0001 ****).

**Effect of *Rhodosporidium* biomasses on the binding of S. *enterica* serovar Dublin on piglet intestinal epithelium.**

The identities of test products and doses are shown in Figure 2 below the patterns of columns. Hundred % adherence is in the absence of any test products (HEPES-Hanks buffer). Error bars indicate SE between 4 replicate reaction vessels and the number in the base of the column indicates the percentage difference when compared to HEPES. The asterisks in Figure 2 show the statistical significance of the difference to control with no test product according to the Student's t-test (p-value < 005 *, p-value < 0.01 **, p-value < 0.001 ***, p-value < 0.0001 ****).

All of the tested hydrothermally processed and oil extracted microbial biomasses from *Rhodosporidium* grown on pure saccharides or lignocellulosic sugars had similar inhibition patterns both for *E. coli* and S. *enterica.* The results also indicate that the hydrothermal processing must be conducted at temperatures above 140 °C to provide biomass with enhanced pathogen binding properties. All of the tested microbial biomasses, which had been subjected to hydrothermal processing at above 140 °C inhibited both tested pathogens even at low dose more efficiently compared to the biomasses that had not been hydrothermally processed.

Effective components in the thermally harvested yeast are unknown, but it is hypothesized that carbohydrate components in yeast mask pathogen binding receptors on the intestinal epithelium or adhesives on the pathogen surfaces In addition compounds originating from lignocellulose hydrolysates may have additional impacts on decreasing pathogen binding to intestinal epithelium. It is evident that thermal harvesting has an additional effect on decreasing the pathogen binding.

### Example 6

**Preparation of hydrothermally processed *Rhodosporidium* biomass grown on pure saccharides for vitamin and amino acid binding examples**

*Rhodosporidium toruloides* strain C-71034 (or other *R. toruloides* strain, which are readily available from recognized microbial culture collections) was grown under aeration in a pilot fermentor. Fermentation was done as fed-batch fermentation using glucose as carbon source. After 6 h batch phase glucose syrup was added to the fermentor periodically during the 167 h cultivation. Growth medium was supplemented with yeast extract (10 g/l), $(NH_4)_2HPO_4$ (1,5 g/l), $(NH_4)_2SO_4$ (1,5 g/l), $MgCl_2$ (1,8 g/l), $K_2HPO_4$ (1 g/l), $KH_2PO_4$ (3 g/l) and $CaCl_2$ (0,4 g/l) and trace minerals $ZnSO_4$ (0,001 g/l), $CuCl$ (0,001 g/l), $MnCl_2$ (0,05 g/l), $NiSO_4x6H_2O$ (0,001 g/l), $Na_2MoO_4$ (0,0001 g/l).

After cultivation the biomass was harvested by thermal treatment by heating the biomass containing cultivation broth in a 500 liter pilot pressure vessel to 180 C and kept in that temperature for 60 minutes. After the thermal treatment the biomass was separated from the liquid phase by filtration. The biomass was washed using tap-water in the filter. The resulting biomass was dried in an oven, milled and extracted using heptane. Residual solvent was removed by drying the biomass using heating to 50 °C and efficient ventilation. This biomass was used in micronutrient binding tests and is named Mass 9.

### Example 7

**Preparation of hydrothermally processes *Rhodosporidium* biomass grown on lignocellulosic hydrolysate for vitamin and amino acid binding examples**

*Rhodosporidium toruloides* strain CBS 8587 (or other *R. toruloides* strain, which are readily available from recognized microbial culture collections) was grown under aeration in a 10-liter fermentor. Fermentation was done as fed-batch fermentation using lignocellulosic hydrolysate syrup as the carbon source. After 10 h batch fermentation lignocellulose hydrolysate syrup was added to the fermentor periodically during the 95 h cultivation. Growth medium was supplemented with yeast extract (8 g/l), $(NH_4)_2SO_4$ (2,5 g/l), $MgSO_4$ (2,5 g/l), $KH_2PO_4$ (3,5 g/l), $K_2HPO_4$ (1,5 g/l) and $CaCl_2$ (0,1 g/l) and trace minerals $ZnSO_4$ (0,0008 g/l), $CuCl$ (0,00008 g/l), $MnSO_4$ (0,0008 g/l), $FeSO_4$ (0,0004 g/l) and $NaH_2PO_4$ (0,5 g/l). Lignocellulosic hydrolysate used was prepared using a thermo-chemical method from straw.

After cultivation the biomass was harvested by thermal treatment by heating the biomass containing cultivation broth in a 1 liter laboratory pressure vessel to 180 °C and kept in that temperature for 60 minutes. After the thermal treatment the biomass was separated from the liquid phase by filtration. The biomass was washed using tap-water in the filter. The resulting biomass was dried in an oven, milled and extracted using heptane. Residual solvent was removed by drying the biomass using efficient ventilation. This biomass was used in micronutrient binding tests and is named Mass 10.

### Example 8

**Testing for unwanted micronutrient binding by yeast biomasses prepared in examples 6-7**

Test product suspensions were incubated in 50 mM phosphate buffer at pH 6.5. The reaction mixtures were prepared in 2 ml microfuge tubes in triplicates. Fresh small intestinal digesta matrix extracted from the small intestine of swine wasused in the micronutrient binder tests.. Solids were removed from the digesta matrix by centrifugation and the liquid matrix was added on top of the binder products. No amino acids were added to this liquid digesta matrix, but the binding was tested in the naturally occurring amino acid concentration of the digesta matrix. Additionally, 990 $\mu$g/ml of ascorbic acid and 198$\mu$g/ml of both thiamine and niacin were added to the mixture. The reaction mixtures in triplicates were incubated for two hours with gentle shaking at 37°C temperature. Finally, solids (test materials) were removed by centrifugation and supernatant was used for vitamin (thiamine, niacin and ascorbic acid) and amino acid (methionine, lysine, threonine and tryptophan) analyses.

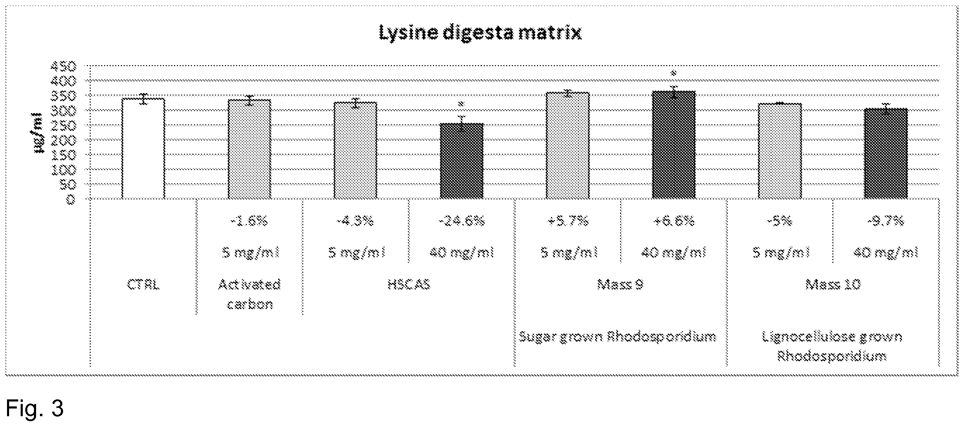

The test products were the biomasses whose production was described in examples 6, 7 and 8. These biomasses are named as Mass 9, Mass 10 in Figures 3, 4, 5, 6, 7, and 8.

The micronutrient binding experiment was carried out in three replicates and in two doses: 5 and 40 mg/ml.

Hydrated sodium calcium aluminosilicate (HSCAS) was used as a positive control in two dose levels and activated carbon in one dose level.

The results were compared to a negative control with no amendment.

**Effect of yeast biomass on the binding of lysine**

In Figure 3 the concentration of free lysine in the presence of potential binders as compared to control with no binder are shown. Error bars indicate standard error (SE) between 3 replicate reaction vessels and asterisks the statistical significance of the difference to control according to the Student's t-test (p-value < 0.05 *, p-value < 0.01 **, p-value < 0.001 ***, p-value < 0.0001 ****). As shown in Figure 3, the test products showed no statistically significant binding of lysine. The positive control did bind lysine at the highest dose applied.

**Effect of yeast biomass on the binding of threonine**

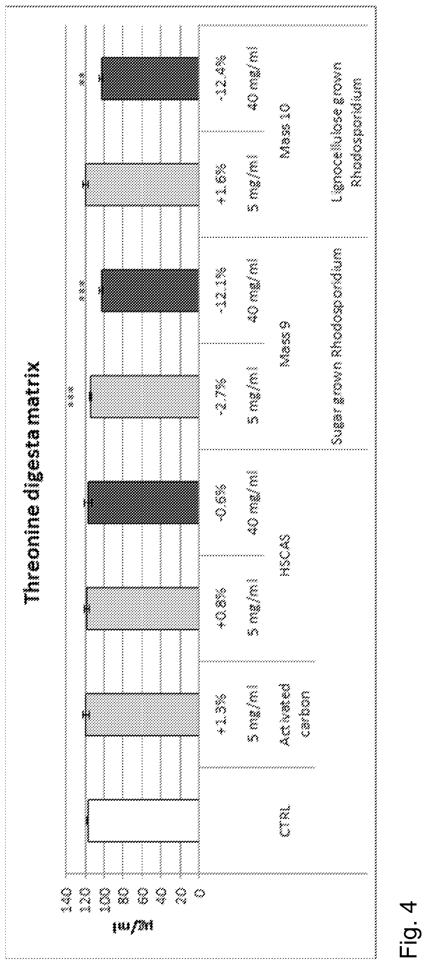

In Figure 4 the concentration of free threonine in the presence of potential binders as compared to control with no binder are shown. Error bars indicate standard error (SE) between 3 replicate reaction vessels and asterisks the statistical significance of the difference to control according to the Student's t-test (p-value < 0.05 *, p-value < 0.01 **, p-value < 0.001 ***, p-value < 0.0001 ****). As shown in Figure 4, the test products bound threonine at the highest dose applied. However, only 11-13% of the threonine was bound by the test products. The positive control did not bind threonine.

**Effect of yeast biomass on the binding of tryptophan**

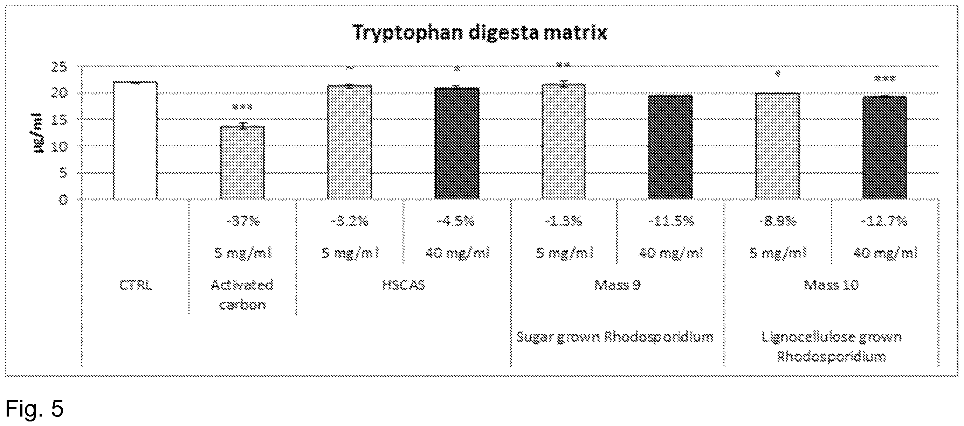

In Figure 5 the concentration of free tryptophan in the presence of potential binders as compared to control with no binder are shown. Error bars indicate standard error (SE) between 3 replicate reaction vessels and asterisks the statistical significance of the difference to control according to the Student's t-test (p-value < 0.05 *, p-value < 0.01 **, p-value < 0.001 ***, p-value < 0.0001 ****). As shown in Figure 5, the test products as well as the positive control bound tryptophan. The amount of tryptophan bound compared to the control was less than 15%.

**Effect of yeast biomass on the binding of methionine**

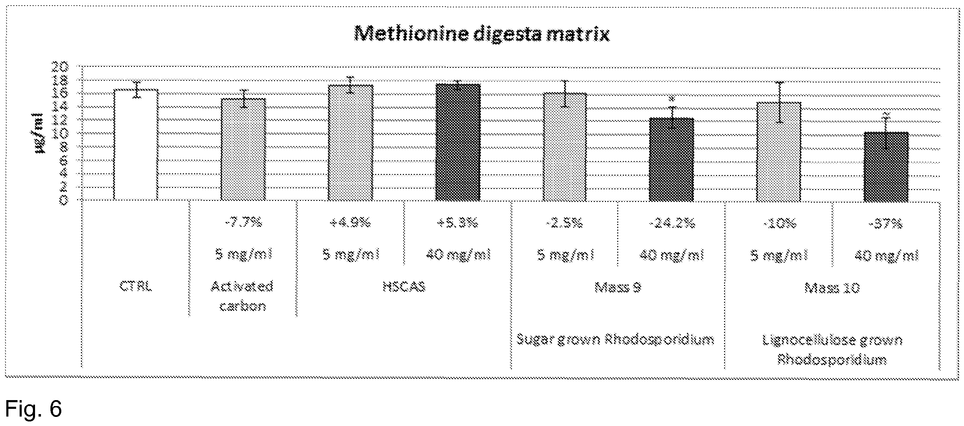

In Figure 6 the concentration of free methionine in the presence of potential binders as compared to control with no binder are shown. Error bars indicate standard error (SE) between 3 replicate reaction vessels and asterisks the statistical significance of the difference to control according to the Student's t-test (p-value < 0.05 *, p-value < 0.01 **, p-value < 0.001 ***, p-value < 0.0001 ****). As shown in Figure 6, none of the test products or the positive control bound methionine.

**Effect of yeast biomass on the binding of thiamine (Vitamin B1)**

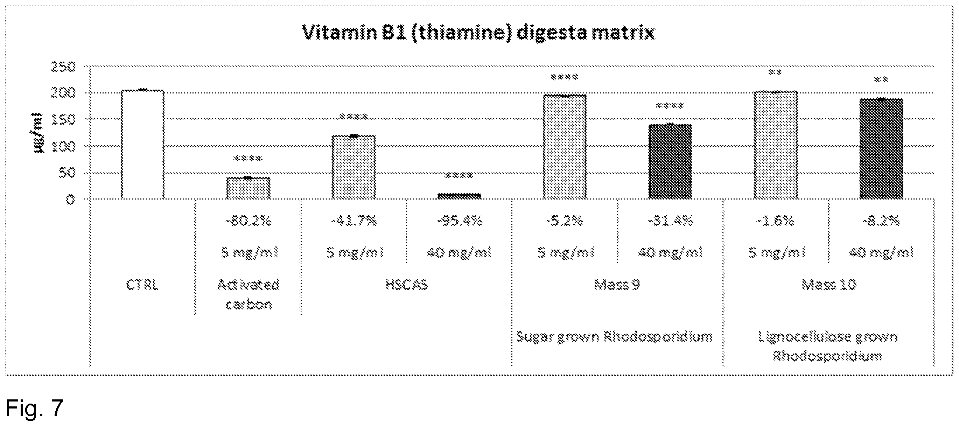

In Figure 7 the concentration of free thiamine in the presence of potential binders as compared to control with no binder are shown. Error bars indicate standard error (SE) between 3 replicate reaction vessels and asterisks the statistical significance of the difference to control according to the Student's t-test (p-value < 0.05 *, p-value < 0.01 **, p-value < 0.001 ***, p-value < 0.0001 ****). As shown in Figure 7, the tested products bound up to one third of the thiamine present at high dosages. However, both positive controls bound over 80% of thiamine at the highest test dose.

**Effect of yeast biomass on the binding of niacin (Vitamin B3)**

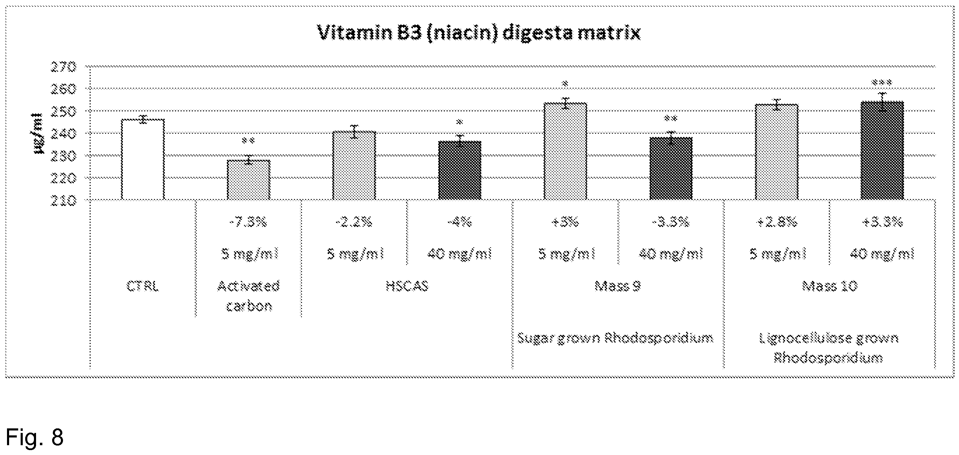

In Figure 8 the concentration of free thiamine in the presence of potential binders as compared to control with no binder are shown. Error bars indicate standard error (SE) between 3 replicate reaction vessels and asterisks the statistical significance of the difference to control according to the Student's t-test (p-value < 0.05 *, p-value < 0.01 **, p-value < 0.001 ***, p-value < 0.0001 ****). As shown in Figure 8, none of the test products bound niacin, on the contrary, some test products increased the concentration of niacin statistically significantly due to niacin present in the products.

The results indicate that none of tested hydrothermally processed biomasses significantly bind the tested vitamins even at high biomass concentrations whereas activated carbon and HSCAS was found to significantly bind the thiamine.

The results also indicate that none of the hydrothermally processed biomasses significantly bind any of the tested amino acids except methionine even at relatively high biomass concentrations whereas HSCAS was found to significantly bind lysine and activated carbon was found to bind tryptophan even at low concentrations.

Generally the results of examples 5 and 9 indicate that hydrothermal processing significantly increases the ability of the microbial biomass to bind pathogens in animal digestive tract but the hydrothermally processed biomass does not significantly bind valuable nutrients such as vitamins and amino acids.

Reference to biomass samples used in the examples

| Name used in the examples | Description | Preparation example | Binding example |
|---|---|---|---|
| Mass 1 | Rhodosporidium Sugar 180°C/60 min not washed | Ex 1 | Ex 5 |
| Mass 2 | Rhodosporidium Sugar 210°C/10 min not washed | Ex 1 | Ex 5 |
| Mass 3 | Rhodosporidium Sugar 140°C/15 min not washed | Ex 1 | Ex 5 |
| Mass 4 | Rhodosporidium Ligno 180°C/60 min washed | Ex 2 | Ex 5 |
| Mass 5 | Rhodosporidium Ligno 180°C/60 min not washed | Ex 2 | Ex 5 |
| Mass 6 | Rhodosporidium Ligno 220°C/20 min washed | Ex 2 | Ex 5 |
| Mass 7 | Rhodosporidium Sugar no thermal harvesting | Ex 3 | Ex 5 |
| Mass 8 | Rhodosporidium Ligno no thermal harvesting | Ex 4 | Ex 5 |
| Mass 9 | Rhodosporidium Sugar 180°C/60min not washed | Ex 6 | Ex 8 |
| Mass 10 | Rhodosporidium Ligno 180°C/60 min washed | Ex 7 | Ex 8 |

**Claims**

1. A method for inactivating a pathogen in a contaminated feed or food product, said method comprising the steps of

    (a) providing a feed or food product contaminated with a pathogen,
    (b) providing a feed or food additive obtained by

        (i) providing an aqueous suspension comprising microbial biomass,
        (ii) subjecting said aqueous suspension comprising said microbial biomass to a temperature of at least 160°C for 1s to 360 minutes at a pressure above 5 bar,
        (iii) separating a solid phase containing the microbial biomass,
        (iv) optionally, drying the microbial biomass obtained from step (iii),

    (c) mixing the feed or food product of (a) with the feed or food additive of (b).

2. The method according to claim 1, wherein step (ii) is conducted under conditions corresponding to a severity factor of at least 2.5, preferably of at least 3.5, most preferably at least 3.9

3. The method of according to any of the preceding claims, wherein said microbial biomass is obtained from *Rhodosporidium, Rhodotorula, Cryptococcus, Lipomyces, Saccharomyces, Aspergillus* or *Mortierella.*

4. The method according to any of the preceding claims, wherein said aqueous suspension comprising microbial biomass contains cultivation medium comprising lignocellulose hydrolysate.

5. The method according to any of the preceding claims, wherein the microbial biomass obtained in step (iii) further comprises residues of lignocellulose.

6. The method of according to any of the preceding claims, wherein the microbial biomass is obtained from oleaginous microorganisms.

7. The method of according to claim 6, wherein the microbial biomass of step (iii) is obtained by subjecting the oleaginous microorganisms to a step of removing the microbial oil from said microbial biomass after step (ii).

8. Hydrothermally processed microbial biomass for use in preventing or reducing the adverse effects of pathogens in animal or human digestive tract, **characterized in that** said hydrothermally processed microbial biomass is obtainable by cultivating microorganism on a cultivation medium and subsequently subjecting microbial biomass from said cultivation to a hydrothermal treatment at a temperature of at least 160 °C for 1s to 360 minutes at a pressure above 5 bar.

9. The biomass for use in preventing or reducing the adverse effects of pathogens in animal or human digestive tract according to claim 8, **characterized in that** the hydrothermal treatment is conducted at a temperature of from 180 °C to 250 °C.

10. The biomass for use in preventing or reducing the adverse effects of pathogens in animal or human digestive tract according to claims 8, **characterized in that** the hydrothermal treatment is conducted in conditions corresponding to a severity factor of at least 2.5, preferably of at least 3.5, most preferably at least 3.9.

11. The biomass for use in preventing or reducing the adverse effects of pathogens in animal or human digestive tract according to any one of claims 8-10, **characterized in that** the cultivation medium comprises lignocellulose hydrolysate.

12. The biomass for use in preventing or reducing the adverse effects of pathogens in animal or human digestive tract according to any one of claims 8-11, **characterized in that** the microbial biomass comprises residues of lignocellulose.

13. The biomass for use in preventing or reducing the adverse effects of pathogens in animal or human digestive tract according to any one of claims 8-12, **characterized in that** the microbial biomass is residual microbial biomass obtained from a single cell oil production process.

14. The biomass for use in preventing or reducing the adverse effects of pathogens in animal or human digestive tract according to any one of claims 8-13, **characterized in that** the microbial biomass is obtained from strains of yeasts belonging to genus *Rhodosporidium, Rhodotorula, Cryptococcus, Lipomyces, Saccharomyces* or to filamentous fungi belonging to genus *Aspergillus* or *Mortierella.*

**Patentansprüche**

1. Verfahren zum Inaktivieren eines Pathogenes in einem kontaminierten Futtermittel- oder Nahrungsmittelprodukt, wobei das Verfahren folgende Schritte umfasst:

   (a) Bereitstellen eines mit einem Pathogen kontaminierten Futtermittel- oder Nahrungsmittelproduktes,
   (b) Bereitstellen eines Futtermittel- oder Nahrungsmittelzusatzes, erhalten durch

   (i) Bereitstellen einer, mikrobielle Biomasse umfassenden, wässrigen Suspension,
   (ii) Unterziehen der, die mikrobielle Biomasse umfassenden, wässrigen Suspension einer Temperatur von mindestens 160 °C für 1 Sekunde bis 360 Minuten bei einem Druck von über 5 Bar,
   (iii) Trennen einer die mikrobielle Biomasse enthaltenden festen Phase,
   (iv) eventuell Trocknen der in Schritt (iii) erhaltenen mikrobiellen Biomasse,

   (c) Mischen des Futtermittel- oder Nahrungsmittelproduktes aus (a) mit dem Futtermittel- oder Nahrungsmittelzusatz aus (b).

2. Verfahren nach Anspruch 1, wobei Schritt (ii) unter Bedingungen, die einem Severity-Faktor von mindestens 2,5, bevorzugt von mindestens 3,5, am meisten bevorzugt mindestens 3,9 entsprechen, ausgeführt wird.

3. Verfahren nach einem der vorgehenden Ansprüche, wobei die mikrobielle Biomasse aus *Rhodosporidium, Rhodotorula, Cryptococcus, Lipomyces, Saccharomyces, Aspergillus* oder *Mortierella* erhalten wird.

4. Verfahren nach einem der vorgehenden Ansprüche, wobei die mikrobielle Biomasse umfassende wässrige Suspension Lignocellulosehydrolysat umfassendes Kultivierungsmedium enthält.

**5.** Verfahren nach einem der vorgehenden Ansprüche, wobei die in Schritt (iii) erhaltene mikrobielle Biomasse ferner Lignocellulosereste umfasst.

**6.** Verfahren of nach einem der vorgehenden Ansprüche, wobei die mikrobielle Biomasse aus ölhaltigen Mikroorganismen erhalten wird.

**7.** Verfahren nach Anspruch 6, wobei die mikrobielle Biomasse aus Schritt (iii) durch Unterziehen der ölhaltigen Mikroorganismen eines Schrittes der Entfernung des mikrobiellen Öls aus der mikrobielle Biomasse nachfolgend Schritt (ii) erhalten wird.

**8.** Hydrothermisch behandelte mikrobielle Biomasse zur Anwendung bei der Vorbeugung oder Reduktion der Nebenwirkungen von Pathogenen im tierischen oder menschlichen Verdauungstrakt, **dadurch gekennzeichnet, dass** die hydrothermisch behandelte mikrobielle Biomasse durch Kultivierung von Mikroorganismen in einem Kultivierungsmedium und nachfolgendes Unterziehen der mikrobiellen Biomasse aus der Kultivierung einer hydrothermischen Behandlung bei einer Temperatur von mindestens 160 °C für 1 Sekunde bis 360 Minuten bei einem Druck von über 5 Bar, erhältlich ist.

**9.** Biomasse zur Anwendung bei der Vorbeugung oder Reduktion der Nebenwirkungen von Pathogenen im tierischen oder menschlichen Verdauungstrakt nach Anspruch 8, **dadurch gekennzeichnet, dass** die hydrothermische Behandlung bei einer Temperatur von 180 °C bis 250 °C ausgeführt wird.

**10.** Biomasse zur Anwendung bei der Vorbeugung oder Reduktion der Nebenwirkungen von Pathogenen im tierischen oder menschlichen Verdauungstrakt nach Anspruch 8, **dadurch gekennzeichnet, dass** die hydrothermische Behandlung unter Bedingungen, die einem Severity-Faktor von mindestens 2,5, bevorzugt mindestens 3,5, am meisten bevorzugt mindestens 3,9 entsprechen, ausgeführt wird.

**11.** Biomasse zur Anwendung bei der Vorbeugung oder Reduktion der Nebenwirkungen von Pathogenen im tierischen oder menschlichen Verdauungstrakt nach einem der Ansprüche 8-10, **dadurch gekennzeichnet, dass** das Kultivierungsmedium Lignocellulosehydrolysat umfasst.

**12.** Biomasse zur Anwendung bei der Vorbeugung oder Reduktion der Nebenwirkungen von Pathogenen im tierischen oder menschlichen Verdauungstrakt nach einem der Ansprüche 8-11, **dadurch gekennzeichnet, dass** die mikrobielle Biomasse Lignocellulosereste umfasst.

**13.** Biomasse zur Anwendung bei der Vorbeugung oder Reduktion der Nebenwirkungen von Pathogenen im tierischen oder menschlichen Verdauungstrakt nach einem der Ansprüche 8-12, **dadurch gekennzeichnet, dass** die mikrobielle Biomasse in einem Herstellungsprozess für einzelliges Öl erhaltene mikrobielle Restbiomasse ist.

**14.** Biomasse zur Anwendung bei der Vorbeugung oder Reduktion der Nebenwirkungen von Pathogenen im tierischen oder menschlichen Verdauungstrakt nach einem der Ansprüche 8-13, **dadurch gekennzeichnet, dass** die mikrobielle Biomasse aus Hefestämmen, die zur Gattung *Rhodosporidium, Rhodotorula, Cryptococcus, Lipomyces, Saccharomyces* gehören, oder filamentösen Pilzen, die zur Gattung *Aspergillus* oder *Mortierella* gehören, erhalten wird.

**Revendications**

**1.** Procédé d'inactivation d'un agent pathogène dans un aliment pour animaux ou un produit alimentaire contaminé, ledit procédé comprenant les étapes consistant à

(a) fournir un aliment pour animaux ou un produit alimentaire contaminé par un agent pathogène,
(b) fournir un additif pour aliment pour animaux ou produit alimentaire obtenu par

(i) la fourniture d'une suspension aqueuse comprenant une biomasse microbienne,
(ii) la soumission de ladite suspension aqueuse comprenant ladite biomasse microbienne à une température d'au moins 160°C pendant 1 seconde à 360 minutes à une pression au-dessus de 5 bar,
(iii) la séparation d'une phase solide contenant la biomasse microbienne,
(iv) éventuellement, le séchage de la biomasse microbienne obtenue de l'étape (iii),

(c) le mélange de l'aliment pour animaux ou du produit alimentaire de (a) avec l'aliment pour animaux ou l'additif alimentaire de (b).

2. Procédé selon la revendication 1, dans lequel l'étape (ii) est effectué dans des conditions correspondant à un facteur de sévérité d'au moins 2,5, préférablement d'au moins 3,5, le plus préférablement d'au moins 3,9

3. Procédé selon l'une quelconque des revendications précédentes, dans lequel ladite biomasse microbienne est obtenue à partir de *Rhodosporidium, Rhodotorula, Cryptococcus, Lipomyces, Saccharomyces, Aspergillus* ou *Mortierella.*

4. Procédé selon l'une quelconque des revendications précédentes, dans lequel ladite suspension aqueuse comprenant de la biomasse microbienne contient un milieu de culture comprenant de l'hydrolysat de lignocellulose.

5. Procédé selon l'une quelconque des revendications précédentes, dans lequel ladite biomasse microbienne obtenue à l'étape (iii) en outre comprend des résidus de lignocellulose.

6. Procédé selon l'une quelconque des revendications précédentes, dans lequel ladite biomasse microbienne est obtenue à partir de micro-organismes oléagineux.

7. Procédé selon la revendication 6, dans lequel la biomasse microbienne de l'étape (iii) est obtenue en soumettant les microorganismes oléagineux à une étape consistant à éliminer l'huile microbienne de ladite biomasse microbienne après l'étape (ii).

8. Biomasse microbienne traitée par hydrothermie destinée à prévenir ou à réduire les effets néfastes d'agents pathogènes dans le tube digestif humain ou animal, **caractérisée en ce que** ladite biomasse microbienne traitée par hydrothermie peut être obtenue en cultivant un microorganisme sur un milieu de culture et en soumettant ensuite la biomasse microbienne de ladite culture à un traitement hydrothermal à une température d'au moins 160 °C pendant 1 seconde à 360 minutes à une pression au-dessus de 5 bar.

9. Biomasse à utiliser pour prévenir ou réduire les effets néfastes d'agents pathogènes dans le tube digestif humain ou animal selon la revendication 8, **caractérisée en ce que** le traitement hydrothermal est effectué à une température allant de 180 °C à 250 °C.

10. Biomasse à utiliser pour prévenir ou réduire les effets néfastes d'agents pathogènes dans le tube digestif humain ou animal selon la revendication 8, **caractérisée en ce que** le traitement hydrothermal est effectué dans des conditions correspondant à un facteur de sévérité d'au moins 2,5, préférablement d'au moins 3,5, le plus préférablement d'au moins 3,9.

11. Biomasse à utiliser pour prévenir ou réduire les effets néfastes d'agents pathogènes dans le tube digestif humain ou animal selon l'une quelconque des revendications 8 à 10, **caractérisée en ce que** le milieu de culture comprend de l'hydrolysat de lignocellulose.

12. Biomasse à utiliser pour prévenir ou réduire les effets néfastes d'agents pathogènes dans le tube digestif humain ou animal selon l'une quelconque des revendications 8 à 11, **caractérisée en ce que** la biomasse microbienne comprend des résidus de lignocellulose.

13. Biomasse à utiliser pour prévenir ou réduire les effets néfastes d'agents pathogènes dans le tube digestif humain ou animal selon l'une quelconque des revendications 8 à 12, **caractérisée en ce que** la biomasse microbienne est une biomasse microbienne résiduelle obtenue à partir d'un processus de production d'huile à cellule unique.

14. Biomasse à utiliser pour prévenir ou réduire les effets néfastes d'agents pathogènes dans le tube digestif humain ou animal selon l'une quelconque des revendications 8 à 13, **caractérisé en ce que** la biomasse microbienne est obtenue à partir de souches de levures appartenant au genre *Rhodosporidium, Rhodotorula, Cryptococcus, Lipomyces, Saccharomyces* ou à champignons filamenteux appartenant au genre *Aspergillus* ou *Mortierella.*

Fig. 1

Fig. 2

Fig. 3

EP 3 157 539 B1

Fig. 4

**Tryptophan digesta matrix**

Fig. 5

EP 3 157 539 B1

**Methionine digesta matrix**

| | CTRL | Activated carbon | HSCAS | | Mass 9 | | Mass 10 | |
|---|---|---|---|---|---|---|---|---|
| % | | -7.7% | +4.9% | +5.3% | -2.5% | -24.2% | -10% | -37% |
| | | 5 mg/ml | 5 mg/ml | 40 mg/ml | 5 mg/ml | 40 mg/ml | 5 mg/ml | 40 mg/ml |
| | | | | | Sugar grown Rhodosporidium | | Lignocellulose grown Rhodosporidium | |

Fig. 6

**Vitamin B1 (thiamine) digesta matrix**

| | CTRL | Activated carbon | HSCAS | | Mass 9 | | Mass 10 | |
|---|---|---|---|---|---|---|---|---|
| | | -80.2% | -41.7% | -95.4% | -5.2% | -31.4% | -1.6% | -8.2% |
| | | 5 mg/ml | 5 mg/ml | 40 mg/ml | 5 mg/ml | 40 mg/ml | 5 mg/ml | 40 mg/ml |
| | | | | | Sugar grown Rhodosporidium | | Lignocellulose grown Rhodosporidium | |

Fig. 7

EP 3 157 539 B1

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120034344 A1, Menon

- US 20120082759 A

**Non-patent literature cited in the description**

- **KOGAN ; KOCHER.** Role of yeast polysaccharides in pig nutrition and health protection. *Livestock science,* 2007, vol. 109, 161-165
- **KNUTSON et al.** Effects of activated charcoal on binding E. coli O157:H7 and Salmonella typhimurium in sheep. *Small Ruminant Res.,* 2006, vol. 65, 101-105

- **PEDERSEN, M. ; A. S. MEYER.** Lignocellulose pretreatment severity - relating pH to biomatrix opening. *New Biotechnology,* 2010, vol. 27 (6), 739-750